# EUROPEAN PATENT SPECIFICATION

(11) **EP 3 828 155 B1**
(45) Date of publication and mention of the grant of the patent: **12.02.2025**
(21) Application number: 20209292.0
(22) Date of filing: 23.11.2020
(51) Int. Cl.: C07C 2/84, C07C 15/46, B01J 27/24

(54) **PROCESS FOR THE PRODUCTION OF FUNCTIONALISED STYRENE MONOMERS FROM BENZENE OR AROMATIC OXYGENATES**
VERFAHREN ZUR HERSTELLUNG FUNKTIONALISIERTER STYROLMONOMERE AUS BENZOL ODER AROMATISCHEN OXYGENATEN
PROCÉDÉ POUR LA PRÉPARATION DE MONOMERES STYRENIQUES A PARTIR DE BENZÈNE OU COMPOSÉS AROMATIQUES OXYGÉNÉS

(30) Priority: 29.11.2019 LU 101516
(43) Date of publication of application: 02.06.2021
(73) Proprietor: Kemijski Institut, 1000 Ljubljana (SI)
(72) Inventor: Likozar, Blaz, 4000 Kranj (SI); Avasthi, Kalpana, 470 002 Sagar, Madhya Pradesh (IN); Bohre, Ashish, 470 002 Sagar, Madhya Pradesh (IN); Grilc, Miha, 1291 Skofljica (SI)
(74) Representative: Zacco GmbH

(56) References cited:
- EP-A1- 1 941 946
- GB-A- 1 436 467
- US-A- 3 689 583
- US-A- 6 127 590
- FREDERIC GOETTMANN ET AL: "Chemical Synthesis of Mesoporous Carbon Nitrides Using Hard Templates and Their Use as a Metal-Free Catalyst for Friedel-Crafts Reaction of Benzene", ANGEWANDTE CHEMIE INT ED, WILEY-VCH, DE, vol. 45, no. 27, 13 June 2006 (2006-06-13), pages 4467 - 4471, XP002438612, ISSN: 1433-7851, DOI: 10.1002/ANIE.200600412

## Description

This present invention provides a sustainable catalytic process for the production of the functionalised styrene monomers from fossil-based or biomass-derived feedstock materials. Methodology involves the coupling of benzene or a functionalized benzene with ethylene in a broad temperature range of 150-300 °C with pressures, ranging from 5 to 100 bar, over heterogeneous catalyst materials. As opposed to the conventional production process from ethylbenzene by dehydrogenation, aromatic ring carbon is directly coupled with ethylene. This opens the door to functionalising the compounds, such as those, derived from biomass, in addition to benzene.

### SUMMARY OF THE INVENTION

The present invention relates to the developing of a sustainable and catalytic process for functionalised styrene monomers synthesis from benzene or functionalized benzene compounds to reduce the overall production cost and provide benefits to pertinent industries and society. Accordingly the present invention provides a sustainable catalytic process for production of functionalised styrene monomers. The method comprises coupling of the benzene or functionalized benzene with ethylene at temperature range from 150-300 °C and pressure range from 5 to 100 bar over heterogeneous catalysts.

### BACKGROUND OF THE INVENTION

Polymer industries utilize a great portion of non-renewable crude oil-based resources. The global polymer production is 322 metric tons with annual growth of 3-4 % (Nat. Commun., 2018, 9, 1-3). Most commonly used polymers such as polyethylene, polystyrene, polyvinylchloride, and poly-methyl methacrylate have dominated the plastic industries for many decades due to their low cost and high durability. The low biodegradability of such polymers and the dependency of (depleting) fossil reserves have intensified interest in green and sustainable resources for the chemical synthesis of polymers. Lignocellulosic biomass represents a promising resource that can be either use as polymer backbones or deconstructed into aromatic molecules and sugar-based platform chemicals (Chem. Rev.2017.11710, 6834-6880).

Styrene is an industrially important commodity chemical, widely used to produce polystyrene plastic, polyesters, protective coatings, resins, rubbers and other copolymers (RSC Adv., 2014, 4, 57087-57097). The chemical structure of styrene contains an active vinyl group that can be further functionalized for the production of versatile synthetic intermediates. The global production of styrene is 33.1 million tons *per* year with the market value of approximately $43.1 billion.

Industrially styrene is produced by the oxidative dehydrogenation of ethylbenzene. Several mild oxidizing agents such as O₂, CO₂, N₂O and SO₂ have been proposed for a low-temperature oxidative dehydrogenation of ethylbenzene (Catal. Sci. Technol., 2013, 3, 519-526). However, molecular oxygen always causes side reactions that reduce the overall styrene selectivity. Additionally, the over-oxidation of ethylbenzene produces CO*ₓ* which makes this reaction highly explosive and greatly lowers the atomic efficiency of such a feedstock. The SNOW (Snamprogetti/Dow) process is a nice alternative that is competitive; nonetheless, multiple steps and the utilization of ethane in large amounts make this process expensive (RSC Adv., 2016, 6, 32989-32993).

The dehydrogenation of ethylbenzene to styrene by using CO₂ as a soft oxidant is an interesting alternative, which was adopted for the first time by Sugino in 1995 (Appl. Catal., A, 1995, 121, 125-137). Apart from a notable positive impact on the global carbon balance, the utilization of CO₂ can offer several advantages such as the acceleration of reaction rate, acting as a diluent, enhancing the product selectivity and thermodynamic stability. The dehydrogenation of ethylbenzene with CO₂ cannot be performed in the current industrial process because of its contribution to catalyst deactivation. Good performances of high-surface ceria, Fe₂O₃/Al₂O₃, TiO₂-ZrO₂, MnO₂-ZrO₂, MCM-41 and SBA-15-supported CeO₂-ZrO₂ catalysts have been reported in the literature (Catal. Sci. Technol., 2015, 5, 5062-5076). However, the reaction temperature of dehydrogenation over these catalysts is still high (above 400 °C).Therefore, developing an efficient catalyst with a high activity at lower temperatures is still highly desirable. Another concern is that the ethylbenzene utilized in these processes is derived from petroleum. Non-renewable fossil sources such as coal, natural gas and petroleum meet nearly 86% of the world's energy and 96% of basic, commodity and specialty chemical demands. The diminishing reserves of these sources, rising atmospheric CO₂ levels, and socio-economic concerns require a reduction of our dependence on these sources.

An alternative method for the production of styrene is a direct and single-step oxidative coupling of biomass derived benzene and ethylene. Rhodium metal based complexes that catalyze ethylene hydrophenylation by benzene C-H activation followed by ethylene insertion into a metal-phenyl bond have been reported as alternatives to dehydrogenation of ethylbenzene (Science, 2015, 348, 6233, 421-424). However, these prior art catalysts give rise to problems in relation with catalyst removal, side- and by-product removal and furthermore often cannot be reused at all.

US3689583A discloses a method for production of dimethylstyrenes by reacting xylenes, ethylene and oxygen in the presence of a lower aliphatic carboxylate of palladium.

### OBJECT OF THE PRESENT INVENTION

Currently, the majority of (functionalised) styrene monomers in the industry is produced by the multiple-step catalytic dehydrogenation reaction from petroleum-derived ethylbenzene using large feeding streams over the iron oxide-based catalyst. However; these processes are extremely endothermic; they consume 10-times the amount of energy as in the production of similar chemicals. In addition, low atom economy, short catalyst lifetimes and the net emission of greenhouse gases are other drawbacks, associated with this industrial process, while the production is based exclusively on a non-renewable fossil-based resource. Natural resources are being consumed in the industrial production of functionalised styrene monomers; from an environmental and economic standpoint, it is important to recoup them in as great extent as possible. The use of petroleum based raw products is a major drawback, which has to be overcome. It would be desirable to have a simple and practicable synthetic route to produce functionalised styrene monomer on an industrial scale starting from non-petroleum sources. The developed processes would be a major breakthrough, complementing the shift of the world economy from the eventually depleting petrochemical feedstock to biomass-based resources.

In this context it would be advantageous to be able to provide a process as outlined above based on the use of sustainable feedstocks such as benzene and ethylene for the production of styrene. Benzene is a widely available renewable resource that can be produced from lignin. Ethylene can also be produced by the dehydration of bio-ethanol. We have also invented an environmental friendly one step process for the production of functionalised styrene monomers from non-renewable aromatic oxygenate compounds.

The industrial route for functionalised styrene monomers requires harsh reaction conditions (high temperature and pressure) that produce a significant amount of by-products which may be difficult to remove and cause further purification and processing problems. In this context, it would be advantageous if one could provide an efficient synthetic process that can replace the current multiple-step and energy-intensive industrial process of the styrene production with relatively mild operating conditions in order to achieve high selectivity. In the context of the prior art methods disclosed above it would also be advantageous if one could provide a process that required the use of heterogeneous catalyst to overcome the problem of homogenous metal complex catalyst that is expensive, difficult to remove and furthermore often cannot be reused at all.

### SUMMARY OF THE PRESENT INVENTION

The present invention solves the problems outlined above and provides the process as defined in claim 1, as well as the use of the catalyst as defined in claim 11. Preferred embodiments of the process and the use of the catalyst are disclosed in claims 2 to 10 and 12 to 15 respectively, as well as in the following description.

### BRIEF DESCRIPTION OF THE FIGURES

FIG. 1 depicts an example of process for the production of styrene from biomass derived benzene and ethylene.
FIG.2 is a wide range angle X-ray diffraction pattern of the graphitic carbon nitride (g-C₃N₄) and palladium doped graphitic carbon nitride (Pd/g-C₃N₄) catalysts.
FIG.3 Nitrogen adsorption-desorption isotherm and pore size distribution (inset) of the graphitic carbon nitride (g-C₃N₄) and palladium doped graphitic carbon nitride (Pd/g-C₃N₄) catalysts.
FIG.4 High resolution TEM image (a), EDX pattern (b) and EDX line scan on the palladium nanoparticle (c-d) of palladium doped graphitic carbon nitride (Pd/g-C₃N₄) catalyst.
FIG.5 FT-IR spectrum of the graphitic carbon nitride (g-C₃N₄) and palladium doped graphitic carbon nitride (Pd/g-C₃N₄) catalysts.
FIG.6 shows the spectrum high-resolution X-ray photoelectron spectra of palladium doped graphitic carbon nitride (Pd/g-C₃N₄) (a) survey spectrum, (b) C 1s, (c) N 1s and (d) Pd 3d.

### DETAILED DESCRIPTION OF THE INVENTION

The present invention is based on a judiciously designed heterogeneous catalyst for the oxidative coupling of benzene or functionalized benzene and ethyleneto produce functionalized styrene monomer. In most of the reported routes, homogeneous metal complexes have been utilized for the production of such monomers but these catalysts have disadvantages in relation to high production cost, separation, recyclability and disposal. Heterogeneous catalytic systems, on the other hand, offer distinct advantages in terms of cost reduction, high activity and selectivity easy separation and reusability. Another merit of heterogeneous catalysts is their stability to high temperature and pressure.

The present invention accordingly provides a process for the oxidative coupling of bio-based or fossil based feedstocks to functionalized styrene monomer with a heterogeneous catalytic system that offers high selectivity under relatively mild reaction conditions. The heterogeneous catalyst of the present invention is a catalyst which in particular enables production of styrene under relatively mild conditions and furthermore allows an easy separation of the used catalyst from the reaction mixture. In particular it has been shown that the catalysts of the present invention can be reused, in many instances even without any reactivation (for example by reductive treatments to remove any undesired oxidation of the transition metals, if contained in the catalysts or other conventional processes known to the skilled person to re-activate a spent catalyst).

Preferred catalysts according to the invention are listed in the following: graphene oxide (GO), sulfonated graphene oxide (SGO), reduced graphene oxide (rGO), multiwalled carbon nanotubes (CNT), graphitic carbon nitride(g-C₃N₄), sulfonated graphitic carbon nitride (S-g-C₃N₄), palladium doped reduced graphene oxide (Pd/rGO), palladium doped multiwalled carbon nanotubes (Pd/CNT), palladium doped graphitic carbon nitride(Pd/g-C₃N₄), boron-doped graphitic carbon nitride(B/g-C₃N₄, palladium doped sulfonated graphitic carbon nitride (Pd/S-g-C₃N₄), ceria doped sulfonated graphene oxide (Ce/SGO), ceria doped reduced graphene oxide (Ce/rGO), ceria doped multiwalled carbon nanotubes(Ce/CNT), ceria doped graphitic carbon nitride(Ce/g-C₃N₄), ceria doped sulfonated graphitic carbon nitride (Ce-S-g-C₃N₄), ruthenium doped reduced graphene oxide (Ru/rGO), ruthenium doped graphitic carbon nitride (Ru/g-C₃N₄), ruthenium doped multiwalled carbon nanotubes (Ru/CNT), ruthenium doped sulfonated graphitic carbon nitride(Ru/S-g-C₃N₄), platinum doped reduced graphene oxide (Pt/rGO), platinum doped graphitic carbon nitride (Pt/g-C₃N₄), platinum doped multiwalled carbon nanotubes (Pt/CNT) and platinum doped sulfonated graphitic carbon nitride (Pt/S-g-C₃N₄). Preferred catalysts are based on graphitic carbon nitride. Accordingly, preferred catalysts of and to be employed in the present invention comprise a graphitic carbon nitride. It is to be understood that preferred embodiments described for the catalyst as such of course also are applicable to the use of the catalyst in the process of the present invention.

Examples of suitable graphitic carbon nitride based catalysts are palladium doped graphitic carbon nitride, platinum doped graphitic carbon nitride and ruthenium doped graphitic carbon nitride.

The catalysts in accordance with the present invention preferably are graphitic carbon nitride and transition metals (Pd, Pt and Ru) supported on this material. The suitable amount of transition metal in the catalysts of the present invention is in the range of from 1 to 10 weight percent relative to the support. The BET specific surface area of the catalysts of the present invention is generally from 0.01 to 200 m² /g, preferably from 0.1 to 150 m² /g, in particular from 1 to 100 m² /g. The pore volume preferably is between 0.1 to 0.50 cm³ per gram, preferably between 0.15 to 0.40 cm³ per gram. In a preferred embodiment the BET surface is from 0.1 to 150 m², preferably from 1 to 100 m², in combination with a pore volume of between 0.1 to 0.50 cm³ per gram, preferably between 0.15 to 0.40 cm³ per gram. BET specific surface area and pore volume are determined according to the methods disclosed in Lowell, S., Shields, J.E., Thomas, M.A., and Thommes, M., (2012). Characterization of porous solids and powders: surface area, pore size and density (Vol. 16), Springer Science & Business Media.

The catalysts disclosed herein, in particular the graphitic carbon nitride catalytic system disclosed herein has particularly shown efficient activity for oxidative coupling reaction. Without being bound to the following explanation, it may be that the superior and unexpected activity is based on the fact that the surface of graphitic carbon nitride is decorated with several oxygen functionalities, such as epoxy, hydroxyl, carbonyls, and nitride groups which induce beneficial oxidised defect sites and promotes the oxidative coupling of benzene or aromatic oxygenate compounds with ethylene to produce functionalized styrene monomers. In any case, the catalytic system disclosed here shows very high activity, selectivity, higher than other catalysts tested or reported in the literature for the oxidative coupling of benzene or functionalized benzene and ethylene to functionalized styrene monomers.

Accordingly the process in accordance with the present invention comprises the step of converting a starting material, selected among benzene or functionalized benzene and ethylene, preferably obtained from renewable (bio-based) or fossil based resources to functionalized styrene monomers in the presence of a catalyst selected from graphene oxide (GO), sulfonated graphene oxide (SGO), reduced graphene oxide (rGO), multiwalled carbon nanotubes (CNT), graphitic carbon nitride(g-C₃N₄), sulfonated graphitic carbon nitride (S-g-C₃N₄), palladium doped reduced graphene oxide (Pd/rGO), palladium doped multiwalled carbon nanotubes (Pd/CNT), palladium doped graphitic carbon nitride (Pd/g-C₃N₄), boron-doped graphitic carbon nitride(B/g-C₃N₄, palladium doped sulfonated graphitic carbon nitride (Pd/S-g-C₃N₄), ceria doped sulfonated graphene oxide (Ce/SGO), ceria doped reduced graphene oxide (Ce/rGO), ceria doped multiwalled carbon nanotubes (Ce/CNT), ceria doped graphitic carbon nitride(Ce/g-C₃N₄), ceria doped sulfonated graphitic carbon nitride (Ce-S-g-C₃N₄), ruthenium doped reduced graphene oxide (Ru/rGO), ruthenium doped graphitic carbon nitride (Ru/g-C₃N₄), ruthenium doped multiwalled carbon nanotubes (Ru/CNT), ruthenium doped sulfonated graphitic carbon nitride(Ru/S-g-C₃N₄), platinum doped reduced graphene oxide (Pt/rGO), platinum doped graphitic carbon nitride (Pt/g-C₃N₄), platinum doped multiwalled carbon nanotubes (Pt/CNT) and platinum doped sulfonated graphitic carbon nitride (Pt/S-g-C₃N₄).

The oxidative coupling reactions are carried out in presence of redox agent. Preferably, redox agent is selected from one of the following: copper (II) acetate, palladium (II) acetate, manganese (II) acetate, silver acetate and sodium acetate. In some embodiments during the oxidation coupling reaction over palladium doped graphitic carbon nitride catalyst, the palladium(III) of the catalyst is reduced, typically to a palladium(I) state. In order for the reaction to be catalytic with respect to palladium, it is necessary to oxidize the palladium(I) back to palladium (III), and so regenerate the catalyst. In some embodiment, this oxidation achieved using a copper(II) acetate or copper(II) redox agent . The term "copper(II) redox agent," as used herein, pertains to a chemical compound comprising at least one copper atom in the +2 oxidation State. In one embodiment, the copper(II) redox agent has only one copper atom.

In one preferred embodiment, the reaction is carried out neat, without a solvent. In one embodiment, the reaction is more preferably carried out neat, without a solvent, with a stoichiometric excess of the benzene, which acts both as reactant and solvent (reaction medium).

It has been found that the concentration of benzene is at least 0.01 M and not more than 1 M, such as from 0.05M to 0.5M, preferably 0.1M to 0.3 M, such as 0.1M, 0.2M, 0.3M, or 0.4M. The pressure of ethylene typically is in the range of from 5 to 100 bar, preferably from 10 to 70 bar and more preferably from 10 to 40 bar, and in particular 30 bar or less and 15 bar or more, such as 20 bar.

It has been found that the amount of catalyst in the reaction mixture (calculated for a batch reaction, suitable adaptations have to be made for continuous processes) is not less than 10 mass percent and not higher than 150 mass percent with respect to substrate (i.e. the above identified starting material), such as from 25 to 100 mass percent. The process disclosed here typically is carried out at a temperature from 150°C to 300°C, preferably from 200°C to 275°C, more preferably from 225°C to 260°C, such as 250°C.

The reaction time is not critical and may depend from the desired balance of conversion and efficiency as well as selectivity, but generally reaction times of form 30 to 1000 min are suitable, such as from 60 to 600 min, from 60 to 300 min and in embodiments from 80 to 200 min.

The reaction may be carried out in any suitable reactors known to the skilled person, but stainless steel reactors (autoclave) are in particular suitable. However, other materials may also be suitable as reactor materials and it is of course also possible to provide the inner surface of such a reactor, completely or partially with a coating of a material inert towards the reaction, such as a polymer coating, a ceramic coating etc.

As said above, the benzene or functionalized benzene and ethylene is/are fossil-based or biomass-derived.

The functionalized benzene according to this invention is a benzene substituted on the phenyl-ring with 1 to 3 substituents selected from C₁₋₄-alkyl.

. In an embodiment the functionalized benzene is benzene substituted by 1 or 2 substituents selected from C₁₋₄-alkyl.

It is to be understood, that the various process parameters, such as starting material concentration, catalyst amount, temperature, pressure and reaction time (residence time), while being disclosed individually, are considered in the context of the present invention in combination, such as a starting material concentration of from 0.05M to 0.5M, a catalyst amount of from 25 to 100 mass percent, a reaction temperature of from 200°C to 275°C, preferably from 225°C to 260°C, a pressure of from 10 to 40 bar, preferably from 15 to 30 bar and a reaction time of from 60 to 300 min. However, all other combinations possible from the ranges and preferred embodiments are also comprised by and disclosed in this invention.

In essence, this leads to the invention best described by the following aspects and embodiments:
In a first aspect is provided a process for the production of, optionally functionalized, styrene monomers comprising the steps:
(a) feeding benzene or functionalized benzene and ethylene, as well as a heterogeneous catalyst into a reactor forming a mixture of benzene or functionalized benzene and ethylene (the starting material), and
(b) heating said mixture and applying pressure to obtain crude styrene;

wherein the step (b) is carried out at a temperature ranging from and including 150°C to 300 °C; and at a pressure (of ethylene) of between and including 5 bar and 100 bar;
wherein the heterogeneous catalyst is selected from graphene oxide (GO), sulfonated graphene oxide (SGO), reduced graphene oxide (rGO), multiwalled carbon nanotubes (CNT), graphitic carbon nitride(g-C₃N₄), sulfonated graphitic carbon nitride (S-g-C₃N₄), palladium doped reduced graphene oxide (Pd/rGO), palladium doped multiwalled carbon nanotubes (Pd/CNT), palladium doped graphitic carbon nitride (Pd/g-C₃N₄), boron-doped graphitic carbon nitride(B/g-C₃N₄, palladium doped sulfonated graphitic carbon nitride (Pd/S-g-C₃N₄), ceria doped sulfonated graphene oxide (Ce/SGO), ceria doped reduced graphene oxide (Ce/rGO), ceria doped multiwalled carbon nanotubes (Ce/CNT), ceria doped graphitic carbon nitride(Ce/g-C₃N₄), ceria doped sulfonated graphitic carbon nitride (Ce-S-g-C₃N₄), ruthenium doped reduced graphene oxide (Ru/rGO), ruthenium doped graphitic carbon nitride (Ru/g-C₃N₄), ruthenium doped multiwalled carbon nanotubes (Ru/CNT), ruthenium doped sulfonated graphitic carbon nitride(Ru/S-g-C₃N₄), platinum doped reduced graphene oxide (Pt/rGO), platinum doped graphitic carbon nitride (Pt/g-C₃N₄), platinum doped multiwalled carbon nanotubes (Pt/CNT) and platinum doped sulfonated graphitic carbon nitride (Pt/S-g-C₃N₄); and
wherein the functionalized benzene is a benzene substituted on the phenyl-ring with 1 to 3 substituents selected from C₁₋₄-alkyl.

In an embodiment the ethylene is added as a gas to the reactor already being fed with the benzene or functionalized benzene and the heterogeneous catalyst.

In another embodiment the phenyl-ring of the functionalized benzene is substituted by 1 or 2 substituents selected from C₁₋₄-alkyl.

In another embodiment the phenyl-ring of the functionalized benzene is substituted by 1 or 2 CH₃-groups.

In another embodiment the, optionally functionalized, styrene monomer is selected from styrene, 2-vinylphenol, 2-methoxy-3-vinylphenol, or 4-methyl-3-vinylphenol.

In another embodiment no solvent is used in the reaction step (b) or added in step (a) in addition to the benzene or the functionalized benzene; preferably while the benzene acts both as reactant and solvent and/or is added in step (a) in stoichiometric excess in relation to the ethylene.

In another embodiment the pressure (of ethylene) in step (b) is between and including 10 bar and 70 bar; and/or
wherein the step (b) is carried out at a temperature ranging from and including 150°C to 250 °C; and/or
wherein the step (b) is carried out for a time period ranging (reaction time / residence time or measured from the time the temperature inside the reactor reaches the intended or given temperature) from and including 60 to 600 minutes.

In another embodiment a redox agent is added to the mixture of step (a) before step (b).

In another embodiment the heterogeneous catalyst is selected from palladium doped sulfonated graphitic carbon nitride (Pd/S-g-C₃N₄), palladium doped graphitic carbon nitride (Pd/g-C₃N₄), ruthenium doped graphitic carbon nitride (Ru/g-C₃N₄), ruthenium doped sulfonated graphitic carbon nitride(Ru/S-g-C₃N₄), platinum doped graphitic carbon nitride (Pt/g-C₃N₄), and platinum doped sulfonated graphitic carbon nitride (Pt/S-g-C₃N₄).

In another embodiment the heterogeneous catalyst is selected from palladium doped graphitic carbon nitride (Pd/g-C3N4) and platinum doped graphitic carbon nitride (Pt/g-C3N4).

In a second aspect is provided the use of a heterogeneous catalyst based on or comprising graphitic carbon nitride for the production of, optionally functionalized, styrene from benzene or functionalized benzene with ethylene; wherein the functionalized benzene is a benzene substituted on the phenyl-ring with 1 to 3 substituents selected from C₁₋₄-alkyl.

In an embodiment the heterogeneous catalyst is a catalyst comprising at least one transition metal or noble metal supported on graphitic carbon nitride.

In another embodiment the heterogeneous catalyst is selected from palladium doped sulfonated graphitic carbon nitride (Pd/S-g-C₃N₄), palladium doped graphitic carbon nitride (Pd/g-C₃N₄), ruthenium doped graphitic carbon nitride (Ru/g-C₃N₄), ruthenium doped sulfonated graphitic carbon nitride(Ru/S-g-C₃N₄), platinum doped graphitic carbon nitride (Pt/g-C₃N₄), and platinum doped sulfonated graphitic carbon nitride (Pt/S-g-C₃N₄); preferably
wherein the heterogeneous catalyst is selected from palladium doped graphitic carbon nitride (Pd/ g-C₃N₄), platinum doped graphitic carbon nitride (Pt/ g-C₃N₄) and ruthenium doped graphitic carbon nitride (Ru/ g-C₃N₄); more preferably
wherein the heterogeneous catalyst is selected from palladium doped graphitic carbon nitride (Pd/g-C₃N₄) and platinum doped graphitic carbon nitride (Pt/g-C₃N₄).

In another embodiment the heterogeneous catalyst comprises the least one transition metal or noble metal in an amount of 1.5 to 15 wt %; and/or wherein the catalyst has a BT surface area of from 0.01 to 200 m² and a pore volume of from 0.1 to 0.50 cm³.

In another embodiment the production of, optionally functionalized, styrene from benzene or functionalized benzene with ethylene is done by the process according to the first aspect of the invention.

### EXAMPLES

In the examples described herein the catalytic oxidative coupling of benzene or with ethylene to styrene monomer was performed using a stainless steel high pressure reactor under the standard operation conditions. The novel process of the present invention was carried out at a temperature greater than 150 °C, most preferably between 150 °C and 300 °C, especially between 200°C and 275°C. The reactions were carried out under pressure. Preferably, the processes are carried out at pressures of at least 10 bar of ethylene, more preferably at least 20 bar, in some embodiments at least 30 bar, in some embodiments pressure is in the range of 10 bar to 70 bar. In general, the reaction should be conducted under conditions where the residence time of the feedstock solution over the catalyst is appropriate to generate the desired products. Preferably, the reaction time is in the range of between 60 and 500 minutes, typically 60-300 minutes, more preferably 60-250 minutes, most preferably 80-200 minutes.

### EXAMPLE 1 Solid Base Catalyst

### Materials

Benzene, ethylene, copper (II) acetate, urea, palladium nitrate, ammonium hydroxide, palladium (II) acetate, manganese (II) acetate, silver acetate, sodium acetate, graphite powder, hydrochloric acid (37%), ethanol, ether, phosphoric acid, hydrogen peroxide (30%), and sulfuric acid, palladium nitrate, platinum nitrate and ruthenium chloride were purchased from Sigma-Aldrich.

### Catalyst Synthesis

Grephitic carbon nitride (g-C₃N₄) catalyst was synthesized by heating 10 g of urea in air at a ramp rate of 2.3 K min⁻¹ to a given temperature (923 K), keeping that temperature for 4 h, then cooling without temperature control. Palladium doped Grephitic carbon nitride (Pd/g-C₃N₄) catalyst was synthesized by incipient-wetness impregnation method. In a typical procedure, 1 g of g-C₃N₄ support was added to 20 mL aqueous solution of Pd(NO₃)₂. The pH of the solution was adjusted to 9 by adding 0.2 M NH₄OH under vigorous stirring. The mixture was then stirred for 1 h at 60 °C. After cooling to room temperature, the solid was recovered by filtration and washed with distilled water. The mixture was then placed in a vacuum oven and allowed to dry overnight at 40 °C. The dried material was then transferred to a Schwartz-type drying tube and reduced in a H₂ flow at 350°C for 3 h. The Pd/g-C₃N₄ catalyst was subsequently cooled to RT under flowing N₂.

### Instrumentation

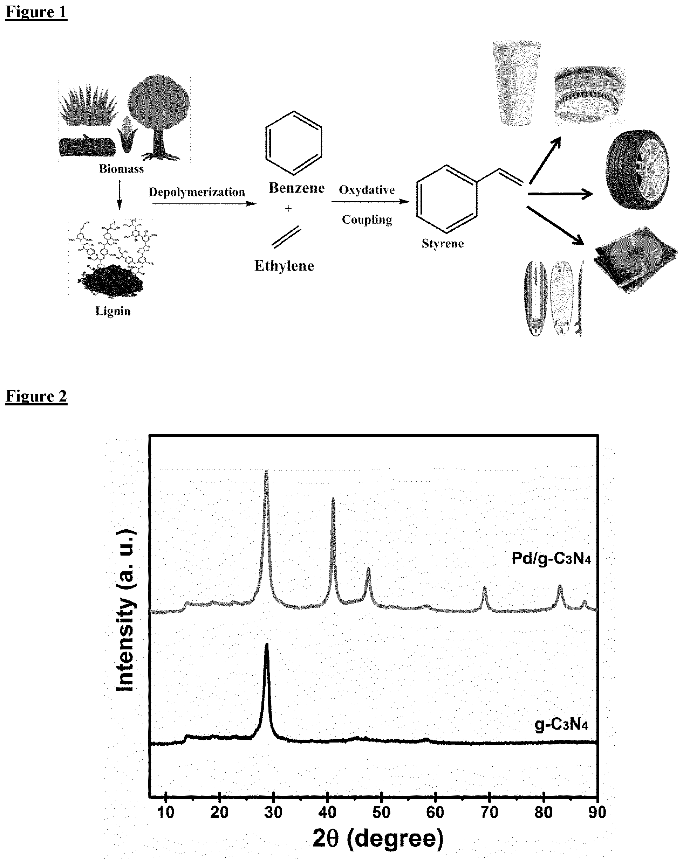

Powder X-ray diffraction (XRD) studies were conducted using the PANalytical X'Pert Pro instrument. Scanning from 10 to 90° was carried out using the CuKα radiation source with the wavelength of 0.15406 nm. Nitrogen physisorption analyses were carried out by degassing the catalysts under N2 flow for 4 h at 200°C. The degassed samples were analyzed in the Micromeritics ASAP 2020 multi-point surface area and porosity analyzer (Figure 2).

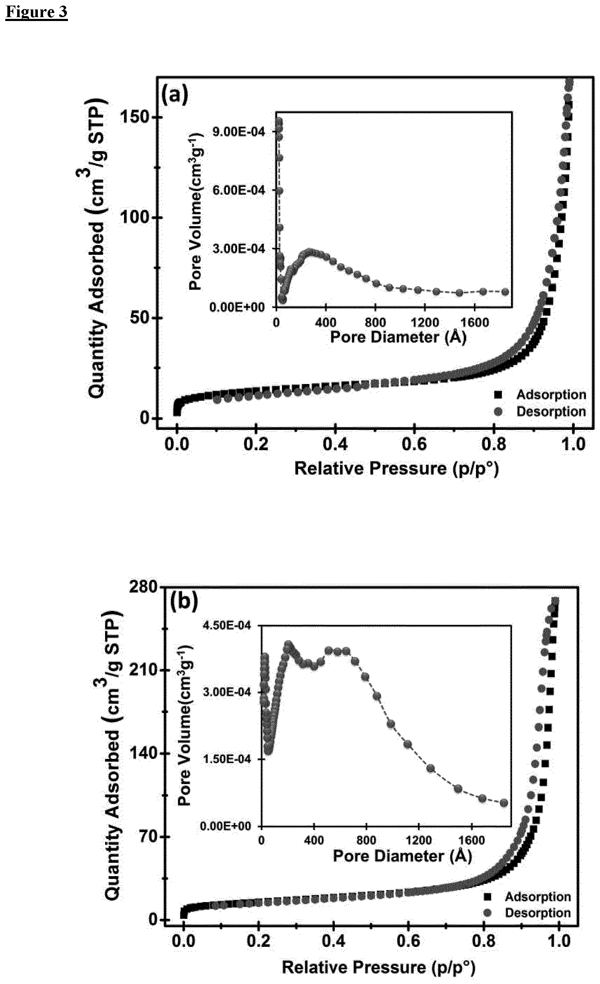

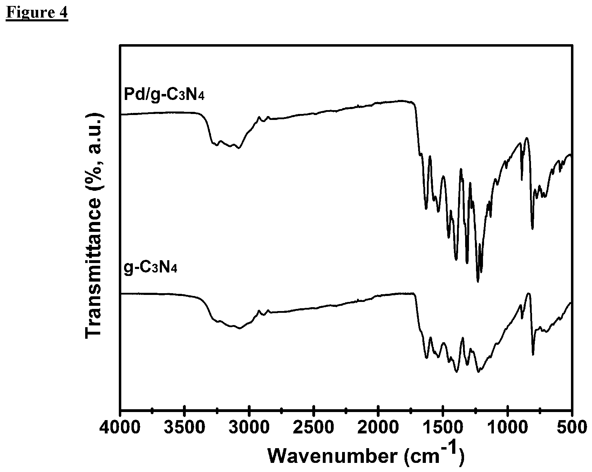

Temperature programmed desorption (TPD) was performed using the Micromeritics 2920 Autochem II Chemisorption Analyzer (Figure 3). The catalysts were pre-treated at 350 °C under the stream of helium for 60 min. The temperature was consequently decreased to 80 °C. 9.8 mol. % CO₂ in He was passed over the catalysts at the flow rate of 30 mL min⁻¹ for 60 min. The excess gas was removed by purging with helium for 30 min. The temperature was after that gradually raised to 900 °C by ramping at 10 °C min⁻¹ under the flow of helium, wherein the desorption data of NH₃, is recorded. The structure and morphology of the prepared catalysts was studied using transmission emission scanning electron microscope (TEM) (Carl Zeiss, Supra 35VP), equipped with energy-dispersive X-ray spectroscopy (EDX) hardware (Oxford Instruments, INCA 400) (Figure 4).

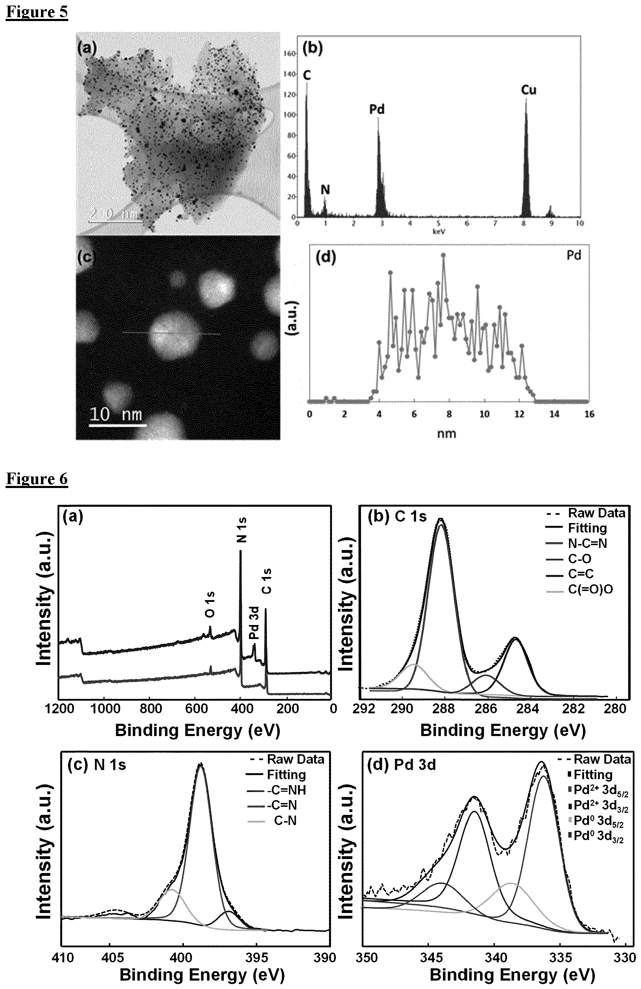

The Fourier-transform infrared (FT-IR) spectra of the samples were recorded on Spectrum Two FT-IR spectrometer (PerkinElmer, Waltham, USA) at the wavenumbers ranging from 4000 cm⁻¹ to 400 cm⁻¹(Figure 5).

X-ray photoelectron spectroscopy (XPS) analyses were carried out by the PHI XPS spectrometer (Physical Electronics). Sample was deposited on adhesive carbon tape and introduced into ultra-high vacuum spectrometer. The vacuum during XPS analyses was in the range of 10⁻⁹ mbar, as a high surface sensitivity is a general characteristic of the XPS methods. Sample surfaces were excited by the X-ray radiation from the monochromatic Al source at the photon energy of 2.3818 × 10⁻¹⁶ J (1486.6 eV). High-energy resolution spectra were acquired with the energy analyser, operating at the resolution of about 9 × 10⁻²⁰ J (0.6 eV) and the pass energy of 4.65 × 10⁻¹⁸ J (29 eV). During data processing, surface spectra were aligned by setting the C 1s peak at 4.57 × 10⁻¹⁷ J (285.0 eV), the latter being characteristic for C-C bonds. The accuracy of binding energies was about ±5 × 10⁻²⁰ J (±0.3 eV). XPS spectra were analysed by the MultiPak software, version 8.0 (Figure 6).

### EXAMPLE 2 Catalytic oxidative coupling reactions and analysis

### Oxidative coupling of benzene with ethylene:

Oxidative coupling reaction can be performed in a high pressure batch reactor (e.g. 250 mL, Amar Equipment Pvt. Ltd.) equipped with a thermocouple, pressure gauge, rupture disk, and gas release valve.

In a typical experiment, 500 mg of Cu(OAc)₂ was dissolved in 100 mL of benzene. The solution was loaded into the autoclave reactor with catalyst (1 g) and sealed. The reactor was pressurized with N₂ to 10 bar and vented three times to remove any residual oxygen atmosphere. Finally the reactor was pressurized to 10 bars with ethylene, stirred (600 rpm) and continued heating at 200 °C. The internal pressure reached 30 bar in heating up time of 35 minutes. The recorded reaction time started when the temperature reached the desired set-point. Once the reaction was completed the reactor was cool to room temperature (15 °C). The liquid phase was analysed by GC (FID) and GC-MS at the end of the reaction. The results are summarized in table 1. The conversion (Conv.) refers to the percentage of ethylene that has been converted to anything, as a result of this process. The selectivity of styrene is the mole percentage of styrene retrieved in the product composition.

**TABLE 1**

| **Catalyst** | **Conversion (%)** | **Selectivity (%)** |
|---|---|---|
| g-C₃N₄ | 10 | 2 |
| Pd/g-C₃N₄ | 70 | 50 |
| Reaction conditions: Benzene 100 mL, ethylene 10 bar, catalyst 1g, time 3h, temperature 200°C. | | |

The same procedure and conditions as in Example 2 is applied to a variety of catalysts. The results are given in Table 2.

**TABLE 2**

| **Catalyst** | **Conversion (%)** | **Selectivity (%)** |
|---|---|---|
| GO | 7 | - |
| rGO | 10 | 4 |
| SGO | 6 | 2 |
| CNT | 7 | 3 |
| Pd/rGO | 50 | 7 |
| Pd/SGO | 45 | 4 |
| Pd/CNT | 47 | 3 |
| Pt/rGO | 55 | 8 |
| Pt/SGO | 49 | 9 |
| Pt/CNT | 52 | 10 |
| Reaction conditions: Benzene 100 mL, ethylene 10 bar, catalyst 1g, time 3h, temperature 200°C. | | |

## Claims

1. A process for the production of, optionally functionalized, styrene monomers comprising the steps:
(a) feeding benzene or functionalized benzene and ethylene, as well as a heterogeneous catalyst into a reactor forming a mixture of benzene or functionalized benzene and ethylene (the starting material), and
(b) heating said mixture and applying pressure to obtain crude styrene;
wherein the step (b) is carried out at a temperature ranging from and including 150°C to 300 °C; and at a pressure (of ethylene) of between and including 5 bar and 100 bar;
wherein the heterogeneous catalyst is selected from graphene oxide (GO), sulfonated graphene oxide (SGO), reduced graphene oxide (rGO), multiwalled carbon nanotubes (CNT), graphitic carbon nitride(g-C₃N₄), sulfonated graphitic carbon nitride (S-g-C₃N₄), palladium doped reduced graphene oxide (Pd/rGO), palladium doped multiwalled carbon nanotubes (Pd/CNT), palladium doped graphitic carbon nitride (Pd/g-C₃N₄), boron-doped graphitic carbon nitride(B/g-C₃N₄, palladium doped sulfonated graphitic carbon nitride (Pd/S-g-C₃N₄), ceria doped sulfonated graphene oxide (Ce/SGO), ceria doped reduced graphene oxide (Ce/rGO), ceria doped multiwalled carbon nanotubes (Ce/CNT), ceria doped graphitic carbon nitride(Ce/g-C₃N₄), ceria doped sulfonated graphitic carbon nitride (Ce-S-g-C₃N₄), ruthenium doped reduced graphene oxide (Ru/rGO), ruthenium doped graphitic carbon nitride (Ru/g-C₃N₄), ruthenium doped multiwalled carbon nanotubes (Ru/CNT), ruthenium doped sulfonated graphitic carbon nitride(Ru/S-g-C₃N₄), platinum doped reduced graphene oxide (Pt/rGO), platinum doped graphitic carbon nitride (Pt/g-C₃N₄), platinum doped multiwalled carbon nanotubes (Pt/CNT) and platinum doped sulfonated graphitic carbon nitride (Pt/S-g-C₃N₄); and
wherein the functionalized benzene is a benzene substituted on the phenyl-ring with 1 to 3 substituents selected from C₁₋₄-alkyl.

2. The process according to claim 1, wherein the ethylene is added as a gas to the reactor already being fed with the benzene or functionalized benzene and the heterogeneous catalyst.

3. The process according to claim 1 or 2, wherein the phenyl-ring of the functionalized benzene is substituted by 1 or 2 substituents selected from C₁₋₄-alkyl.

4. The process according to claim 1 or 2, wherein the phenyl-ring of the functionalized benzene is substituted by 1 or 2 CH₃-groups.

5. The process according to any one of claims 1 to 4, wherein the, optionally functionalized, styrene monomer is selected from styrene, 2-vinylphenol, 2-methoxy-3-vinylphenol, or 4-methyl-3-vinylphenol.

6. The process according to any one of claims 1 to 5, wherein no solvent is used in the reaction step (b) or added in step (a) in addition to the benzene or the functionalized benzene; preferably while the benzene acts both as reactant and solvent and/or is added in step (a) in stoichiometric excess in relation to the ethylene.

7. The process according to any one of claims 1 to 6, wherein the pressure (of ethylene) in step (b) is between and including 10 bar and 70 bar; and/or
wherein the step (b) is carried out at a temperature ranging from and including 150°C to 250 °C; and/or
wherein the step (b) is carried out for a time period ranging (reaction time / residence time or measured from the time the temperature inside the reactor reaches the intended or given temperature) from and including 60 to 600 minutes.

8. The process according to any one of claims 1 to 7, wherein a redox agent is added to the mixture of step (a) before step (b).

9. The process according to any one of claims 1 to 8, wherein the heterogeneous catalyst is selected from palladium doped sulfonated graphitic carbon nitride (Pd/S-g-C₃N₄), palladium doped graphitic carbon nitride (Pd/g-C₃N₄), ruthenium doped graphitic carbon nitride (Ru/g-C₃N₄), ruthenium doped sulfonated graphitic carbon nitride(Ru/S-g-C₃N₄), platinum doped graphitic carbon nitride (Pt/g-C₃N₄), and platinum doped sulfonated graphitic carbon nitride (Pt/S-g-C₃N₄).

10. The process according to any one of claims 1 to 9, wherein the heterogeneous catalyst is selected from palladium doped graphitic carbon nitride (Pd/g-C3N4) and platinum doped graphitic carbon nitride (Pt/g-C3N4).

11. Use of a heterogeneous catalyst based on or comprising graphitic carbon nitride for the production of, optionally functionalized, styrene from benzene or functionalized benzene with ethylene; wherein the functionalized benzene is a benzene substituted on the phenyl-ring with 1 to 3 substituents selected from C₁₋₄-alkyl.

12. The use according to claim 11, wherein the heterogeneous catalyst is a catalyst comprising at least one transition metal or noble metal supported on graphitic carbon nitride.

13. The use according to any one of claims 11 or 12, wherein the heterogeneous catalyst is selected from palladium doped sulfonated graphitic carbon nitride (Pd/S-g-C₃N₄), palladium doped graphitic carbon nitride (Pd/g-C₃N₄), ruthenium doped graphitic carbon nitride (Ru/g-C₃N₄), ruthenium doped sulfonated graphitic carbon nitride(Ru/S-g-C₃N₄), platinum doped graphitic carbon nitride (Pt/g-C₃N₄), and platinum doped sulfonated graphitic carbon nitride (Pt/S-g-C₃N₄); preferably
wherein the heterogeneous catalyst is selected from palladium doped graphitic carbon nitride (Pd/ g-C₃N₄), platinum doped graphitic carbon nitride (Pt/ g-C₃N₄) and ruthenium doped graphitic carbon nitride (Ru/ g-C₃N₄); more preferably
wherein the heterogeneous catalyst is selected from palladium doped graphitic carbon nitride (Pd/g-C₃N₄) and platinum doped graphitic carbon nitride (Pt/g-C₃N₄).

14. The use according to any one of claims 11 to 13, wherein the heterogeneous catalyst comprises the least one transition metal or noble metal in an amount of 1.5 to 15 wt %; and/or wherein the catalyst has a BT surface area of from 0.01 to 200 m² and a pore volume of from 0.1 to 0.50 cm³.

15. The use according to any of claims 11 to 14, wherein the production of, optionally functionalized, styrene from benzene or functionalized benzene with ethylene is done by the process according to claims 1 to 10.

## Patentansprüche

1. Verfahren zur Herstellung von gegebenenfalls funktionalisierten Styrolmonomeren, umfassend die Folgenden Schritte:
(a) Zuführen von Benzol oder funktionalisiertem Benzol und Ethylen sowie einem heterogenen Katalysator in einen Reaktor, wodurch ein Gemisch aus Benzol oder funktionalisiertem Benzol und Ethylen (dem Ausgangsmaterial) gebildet wird, und
(b) Erhitzen des Gemisches und Ausüben von Druck, um ein rohes Styrol zu erhalten;
wobei der Schritt (b) bei einer Temperatur im Bereich von und einschließlich 150°C bis 300 °C und bei einem Druck (des Ethylens) zwischen und einschließlich 5 Bar und 100 Bar ausgeführt wird,
wobei der heterogene Katalysator aus Folgendem ausgewählt ist: Graphenoxid (GO), sulfoniertem Graphenoxid (SGO), reduziertem Graphenoxid (rGO), mehrwandigen Kohlenstoffnanoröhren (CNT), graphitischem Kohlenstoffnitrid (g-C₃N₄), sulfoniertem graphitischem Kohlenstoffnitrid (S-g-C₃N₄), palladiumdotiertem reduziertem Graphenoxid (Pd/rGO), palladiumdotierten mehrwandigen Kohlenstoffnanoröhren (Pd/CNT), palladiumdotiertem graphitischem Kohlenstoffnitrid (Pd/g-C₃N₄), bordotiertem graphitischem Kohlenstoffnitrid (B/g-C₃N₄), palladiumdotiertem sulfoniertem graphitischem Kohlenstoffnitrid (Pd/S-g-C₃N₄), cerdioxiddotiertem sulfoniertem Graphenoxid (Ce/SGO), cerdioxiddotiertem reduziertem Graphenoxid (Ce/rGO), cerdioxiddotierten mehrwandigen Kohlenstoffnanoröhren (Ce/CNT), cerdioxiddotiertem graphitischem Kohlenstoffnitrid (Ce/g-C₃N₄), cerdioxiddotiertem sulfoniertem graphitischem Kohlenstoffnitrid (Ce-S-g-C₃N₄), rutheniumdotiertem reduziertem Graphenoxid (Ru/rGO), rutheniumdotiertem graphitischem Kohlenstoffnitrid (Ru/g-C₃N₄), rutheniumdotierten mehrwandigen Kohlenstoffnanoröhren (Ru/CNT), rutheniumdotiertem sulfoniertem graphitischem Kohlenstoffnitrid (Ru/S-g-C₃N₄), platindotiertem reduziertem Graphenoxid (Pt/rGO), platindotiertem graphitischem Kohlenstoffnitrid (Pt/g-C₃N₄), platindotierten mehrwandigen Kohlenstoffnanoröhren (Pt/CNT) und platindotiertem sulfoniertem graphitischem Kohlenstoffnitrid (Pt/S-g-C₃N₄); und
wobei das funktionalisierte Benzol ein Benzol ist, das an dem Phenylring mit 1 bis 3
Substituenten substituiert ist, die aus C₁₋₄-Alkyl ausgewählt sind.

2. Verfahren nach Anspruch 1, wobei das Ethylen als Gas zu dem Reaktor hinzugefügt wird, dem bereits das Benzol oder funktionalisierte Benzol und der heterogene Katalysator zugeführt wird.

3. Verfahren nach Anspruch 1 oder 2, wobei der Phenylring des funktionalisierten Benzols mit 1 oder 2 Substituenten substituiert ist, die aus C₁₋₄-Alkyl ausgewählt sind.

4. Verfahren nach Anspruch 1 oder 2, wobei der Phenylring des funktionalisierten Benzols mit 1 oder 2 CH₃-Gruppen substituiert ist.

5. Verfahren nach einem der Ansprüche 1 bis 4, wobei das gegebenenfalls substituierte Styrolmonomer aus Styrol, 2-Vinylphenol, 2-Methoxy-3-vinylphenol oder 4-Methyl-3 - vinylphenol ausgewählt ist.

6. Verfahren nach einem der Ansprüche 1 bis 5, wobei kein Lösungsmittel in dem Reaktionsschritt (b) verwendet oder in Schritt (a) zusätzlich zu dem Benzol oder funktionalisierten Benzol hinzugefügt wird; während das Benzol vorzugsweise sowohl als Reaktant als auch als Lösungsmittel fungiert und/oder in Schritt (a) in stöchiometrischem Überschuss in Bezug auf das Ethylen hinzugefügt wird.

7. Verfahren nach einem der Ansprüche 1 bis 6, wobei der Druck (des Ethylens) in Schritt (b) zwischen und einschließlich 10 Bar und 70 Bar liegt und/oder
wobei der Schritt (b) bei einer Temperatur im Bereich von und einschließlich 150 °C bis 250 °C ausgeführt wird und/oder
wobei der Schritt (b) für einen Zeitraum ausgeführt wird, der im Bereich von und einschließlich 60 bis 600 Minuten liegt (Reaktionszeit/Verweilzeit oder gemessen von dem Zeitpunkt, zu dem die Temperatur im Innern des Reaktors die vorgesehene oder gegebene Temperatur erreicht).

8. Verfahren nach einem der Ansprüche 1 bis 7, wobei ein Redoxmittel dem Gemisch aus Schritt (a) vor Schritt (b) hinzugefügt wird.

9. Verfahren nach einem der Ansprüche 1 bis 8, wobei der heterogene Katalysator ausgewählt ist aus palladiumdotiertem sulfoniertem graphitischem Kohlenstoffnitrid (Pd/S-g-C₃N₄), palladiumdotiertem graphitischem Kohlenstoffnitrid (Pd/g-C₃N₄), rutheniumdotiertem graphitischem Kohlenstoffnitrid (Ru/g-C₃N₄), rutheniumdotiertem sulfoniertem graphitischem Kohlenstoffnitrid (Ru/S-g-C₃N₄), platindotiertem graphitischem Kohlenstoffnitrid (Pt/g-C₃N₄) und platindotiertem sulfoniertem graphitischem Kohlenstoffnitrid (Pt/S-g-C₃N₄).

10. Verfahren nach einem der Ansprüche 1 bis 9, wobei der heterogene Katalysator ausgewählt ist aus palladiumdotiertem graphitischem Kohlenstoffnitrid (Pd/g-C₃N₄) und platindotiertem graphitischem Kohlenstoffnitrid (Pt/g-C₃N₄).

11. Verwendung eines heterogenen Katalysators auf Grundlage von graphitischem Kohlenstoffnitrid oder dieses umfassend für die Herstellung von gegebenenfalls funktionalisiertem Styrol aus Benzol oder funktionalisiertem Benzol mit Ethylen; wobei das funktionalisierte Benzol ein Benzol ist, das an dem Phenylring mit 1 bis 3 Substituenten substituiert ist, die aus C₁₋₄-Alkyl ausgewählt sind.

12. Verwendung nach Anspruch 11, wobei der heterogene Katalysator ein Katalysator ist, der mindestens ein Übergangsmetall oder Edelmetall umfasst, das auf graphitischem Kohlenstoffnitrid gestützt wird.

13. Verwendung nach einem der Ansprüche 11 oder 12, wobei der heterogene Katalysator ausgewählt ist aus palladiumdotiertem sulfoniertem graphitischem Kohlenstoffnitrid (Pd/S-g-C₃N₄), palladiumdotiertem graphitischem Kohlenstoffnitrid (Pd/g-C₃N₄), rutheniumdotiertem graphitischem Kohlenstoffnitrid (Ru/g-C₃N₄), rutheniumdotiertem sulfoniertem graphitischem Kohlenstoffnitrid (Ru/S-g-C₃N₄), platindotiertem graphitischem Kohlenstoffnitrid (Pt/g-C₃N₄) und platindotiertem sulfoniertem graphitischem Kohlenstoffnitrid (Pt/S-g-C₃N₄); wobei vorzugsweise
der heterogene Katalysator ausgewählt ist aus palladiumdotiertem graphitischem Kohlenstoffnitrid (Pd/ g-C₃N₄), platindotiertem graphitischem Kohlenstoffnitrid (Pt/ g-C₃N₄) und rutheniumdotiertem graphitischem Kohlenstoffnitrid (Ru/ g-C₃N₄); wobei noch bevorzugter
der heterogene Katalysator ausgewählt ist aus palladiumdotiertem graphitischem Kohlenstoffnitrid (Pd/g-C₃N₄) und platindotiertem graphitischem Kohlenstoffnitrid (Pt/g-C₃N₄).

14. Verwendung nach einem der Ansprüche 11 bis 13, wobei der heterogene Katalysator das mindestens ein Übergangsmetall oder Edelmetall in einer Menge von 1,5 bis 15 Gew.-% umfasst; und/oder wobei der Katalysator einen BT-Oberflächenbereich von 0,01 bis 200 m² und ein Porenvolumen von 0,1 bis 0,50 cm³ aufweist.

15. Verfahren nach einem der Ansprüche 11 bis 14, wobei die Herstellung von gegebenenfalls funktionalisiertem Styrol aus Benzol oder funktionalisiertem Benzol mit Ethylen durch das Verfahren nach den Ansprüchen 1 bis 10 vorgenommen wird.

## Revendications

1. Procédé de production de monomères de styrène éventuellement fonctionnalisés comprenant les étapes suivantes :
(a) l'introduction de benzène ou de benzène et d'éthylène fonctionnalisés, ainsi que d'un catalyseur hétérogène dans un réacteur formant un mélange de benzène ou de benzène et d'éthylène fonctionnalisés (le matériau de départ), et
(b) le chauffage dudit mélange et l'application d'une pression pour obtenir du styrène brut ;
dans lequel l'étape (b) est réalisée à une température comprise entre 150 °C et 300 °C et à une pression (d'éthylène) comprise entre 5 bars et 100 bars ;
dans lequel le catalyseur hétérogène est choisi parmi l'oxyde de graphène (GO), l'oxyde de graphène sulfoné (SGO), l'oxyde de graphène réduit (rGO), les nanotubes de carbone multiparois (CNT), le nitrure de carbone graphitique (g-C₃N₄), le nitrure de carbone graphitique sulfoné (S-g-C₃N₄), l'oxyde de graphène réduit dopé au palladium (Pd/rGO), les nanotubes de carbone multiparois dopés au palladium (Pd/CNT), le nitrure de carbone graphitique dopé au palladium (Pd/g-C₃N₄), le nitrure de carbone graphitique dopé au bore (B/g-C₃N₄, le nitrure de carbone graphitique sulfoné dopé au palladium (Pd/S-g-C₃N₄), l'oxyde de graphène sulfoné dopé à la cérine (Ce/SGO), l'oxyde de graphène réduit dopé à la cérine (Ce/rGO), les nanotubes de carbone multiparois dopés à la cérine (Ce/CNT), le nitrure de carbone graphitique dopé à la cérine (Ce/g-C₃N₄), le nitrure de carbone graphitique sulfoné dopé à la cérine (Ce-S-g-C₃N₄), l'oxyde de graphène réduit dopé au ruthénium (Ru/rGO), le nitrure de carbone graphitique dopé au ruthénium (Ru/g-C₃N₄), les nanotubes de carbone multiparois dopés au ruthénium (Ru/CNT), le nitrure de carbone graphitique sulfoné dopé au ruthénium (Ru/S-g-C₃N₄), l'oxyde de graphène réduit dopé au platine (Pt/rGO), le nitrure de carbone graphitique dopé au platine (Pt/g-C₃N₄), les nanotubes de carbone multiparois dopés au platine (Pt/CNT) et le nitrure de carbone graphitique sulfoné dopé au platine (Pt/S-g-C₃N₄,) ; et
dans lequel le benzène fonctionnalisé est un benzène substitué sur le cycle phényle par 1 à 3 substituants choisis parmi l'alkyle C₁₋₄.

2. Procédé selon la revendication 1, dans lequel l'éthylène est ajouté sous forme de gaz au réacteur déjà alimenté avec le benzène ou le benzène fonctionnalisé et le catalyseur hétérogène.

3. Procédé selon la revendication 1 ou 2, dans lequel le cycle phényle du benzène fonctionnalisé est substitué par 1 ou 2 substituants choisis parmi l'alkyle C₁₋₄.

4. Procédé selon la revendication 1 ou 2, dans lequel le cycle phényle du benzène fonctionnalisé est substitué par 1 ou 2 groupes CH₃.

5. Procédé selon l'une quelconque des revendications 1 à 4, dans lequel le monomère de styrène éventuellement fonctionnalisé est choisi parmi le styrène, le 2-vinylphénol, le 2-méthoxy-3-vinylphénol ou le 4-méthyl-3-vinylphénol.

6. Procédé selon l'une quelconque des revendications 1 à 5, dans lequel aucun solvant n'est utilisé dans l'étape de réaction (b) ou ajouté dans l'étape (a) en plus du benzène ou du benzène fonctionnalisé ; de préférence, le benzène agissant à la fois comme réactif et comme solvant et/ou étant ajouté dans l'étape (a) en excès stœchiométrique par rapport à l'éthylène.

7. Procédé selon l'une quelconque des revendications 1 à 6, dans lequel la pression (d'éthylène) dans l'étape (b) est comprise entre 10 bars et 70 bars ; et/ou
dans lequel l'étape (b) est réalisée à une température comprise entre 150 °C et 250 °C ; et/ou
dans lequel l'étape (b) est réalisée pendant une durée comprise entre 60 et 600 minutes (temps de réaction/temps de séjour ou mesuré à partir du moment où la température à l'intérieur du réacteur atteint la température prévue ou donnée).

8. Procédé selon l'une quelconque des revendications 1 à 7, dans lequel un agent redox est ajouté au mélange de l'étape (a) avant l'étape (b).

9. Procédé selon l'une quelconque des revendications 1 à 8, dans lequel le catalyseur hétérogène est choisi parmi le nitrure de carbone graphitique sulfoné dopé au palladium (Pd/S-g-C₃N₄), le nitrure de carbone graphitique dopé au palladium (Pd/g-C₃N₄), le nitrure de carbone graphitique dopé au ruthénium (Ru/g-C₃N₄), le nitrure de carbone graphitique sulfoné dopé au ruthénium (Ru/S-g-C₃N₄), le nitrure de carbone graphitique dopé au platine (Pt/g-C₃N₄) et le nitrure de carbone graphitique sulfoné dopé au platine (Pt/S-g-C₃N₄).

10. Procédé selon l'une quelconque des revendications 1 à 9, dans lequel le catalyseur hétérogène est choisi parmi le nitrure de carbone graphitique dopé au palladium (Pd/g-C₃N₄) et le nitrure de carbone graphitique dopé au platine (Pt/g-C₃N₄).

11. Utilisation d'un catalyseur hétérogène à base de ou comprenant du nitrure de carbone graphitique pour la production de styrène éventuellement fonctionnalisé à partir de benzène ou de benzène fonctionnalisé avec de l'éthylène ; dans laquelle le benzène fonctionnalisé est un benzène substitué sur le cycle phényle par 1 à 3 substituants choisis parmi l'alkyle C₁₋₄.

12. Utilisation selon la revendication 11, dans laquelle le catalyseur hétérogène est un catalyseur comprenant au moins un métal de transition ou un métal noble supporté sur du nitrure de carbone graphitique.

13. Utilisation selon l'une quelconque des revendications 11 ou 12, dans laquelle le catalyseur hétérogène est choisi parmi le nitrure de carbone graphitique sulfoné dopé au palladium (Pd/S-g-C₃N₄), le nitrure de carbone graphitique dopé au palladium (Pd/g-C₃N₄), le nitrure de carbone graphitique dopé au ruthénium (Ru/g-C₃N₄), le nitrure de carbone graphitique sulfoné dopé au ruthénium (Ru/S-g-C₃N₄), le nitrure de carbone graphitique dopé au platine (Pt/g-C₃N₄) et le nitrure de carbone graphitique sulfoné dopé au platine (Pt/S-g-C₃N₄) ; de préférence
dans lequel le catalyseur hétérogène est choisi parmi le nitrure de carbone graphitique dopé au palladium (Pd/g-C₃N₄), le nitrure de carbone graphitique dopé au platine (Pt/g-C₃N₄) et le nitrure de carbone graphitique dopé au ruthénium (Ru/g-C₃N₄) ; de préférence encore
dans lequel le catalyseur hétérogène est choisi parmi le nitrure de carbone graphitique dopé au palladium (Pd/g-C₃N₄) et le nitrure de carbone graphitique dopé au platine (Pt/g-C₃N₄).

14. Utilisation selon l'une quelconque des revendications 11 à 13, dans laquelle le catalyseur hétérogène comprend au moins un métal de transition ou un métal noble en une quantité de 1,5 à 15 % en poids ; et/ou dans laquelle le catalyseur a une surface BT de 0,01 à 200 m² et un volume de pores de 0,1 à 0,50 cm³.

15. Utilisation selon l'une quelconque des revendications 11 à 14, dans laquelle la production de styrène éventuellement fonctionnalisé à partir de benzène ou de benzène fonctionnalisé avec de l'éthylène est effectuée par le procédé selon les revendications 1 à 10.
